# EUROPEAN PATENT APPLICATION

(11) **EP 4 563 737 A1**
(43) Date of publication of application: **04.06.2025**
(21) Application number: 22952874.0
(22) Date of filing: 14.12.2022
(51) Int. Cl.: D04H 1/492, D04H 1/495, D04H 1/559, D04H 1/54, A61F 13/15

(54) **NON-WOVEN FABRIC HAVING STRIPE PROTRUSIONS ON SURFACE, AND ABSORPTION PRODUCT THEREOF**

(30) Priority: 28.07.2022 CN 202210897774
(71) Applicant: Xiamen Yanjan New Material Co., Ltd, Xiamen, Fujian 361000 (CN)
(72) Inventor: XIE, Jiquan, Xiamen, Fujian 361000 (CN); CAI, Jixiang, Xiamen, Fujian 361000 (CN); PAN, Jianhua, Xiamen, Fujian 361000 (CN); WU, Danpin, Xiamen, Fujian 361000 (CN)
(74) Representative: Cabinet Chaillot
(86) International application number: PCT/CN2022/138962
(87) International publication number: WO 2024/021441

(57) **Abstract**

Disclosed in the present invention are a non-woven fabric having stripe protrusions on a surface, and an absorption product thereof. The surface of the non-woven fabric has several stripe protrusions which longitudinally extend and are transversely distributed; the stripe protrusions have a height between 0.5mm-3.0mm; a plurality of openings are formed in opening areas formed between the adjacent stripe protrusions; and the average area of each opening is less than 20 mm². The longitudinal stripe protrusions on the surface of the non-woven fabric can guide body fluid in the longitudinal direction of the absorption product to increase a body fluid permeation area and increase the body fluid infiltration speed as well as effectively stop the body fluid from leaking laterally.

## Description

### TECHNICAL FIELD

The present invention relates to the field of non-woven fabrics, in particular to a soft and dry non-woven fabric having stripe protrusions on a surface thereof, and an absorption product using said non-woven fabric, applicable to personal care and infant care.

### BACKGROUND OF THE INVENTION

Non-woven fabrics are widely used on surface layers of disposable hygiene products and are well received by consumers because they are soft and skin-friendly. Along with the consumers' higher requirements for hygiene products, consumers are becoming dissatisfied with the existing planar non-woven fabrics; accordingly, perforated non-woven fabrics for increased permeability have been developed. Openings on a surface of a perforated non-woven fabric can effectively increase the permeability of body fluid, such that the body fluid is rapidly guided to an absorbent layer, and the dryness of an outer hygiene surface layer is increased. CN203841923U (application number 201420140109.0) titled "Three-dimensional Non-woven Fabric with Openings" discloses a three-dimensional (i.e. uneven surface, as opposed to flat and smooth surface) non-woven fabric with openings, wherein convex regions, concave regions and openings are distributed on the three-dimensional non-woven fabric; the uneven concave-convex surface of the three-dimensional non-woven fabric is supported by the compression resistance of high-density fiber regions, and the convex regions and the openings have a height difference of more than 0.2 mm. Therefore, even if body fluid remains on the surfaces of the concave regions, only the convex regions will be in contact with the skin of the user, such that the user does not feel wet or damp; the openings accelerate permeation of the body fluid and reduce the amount of body fluid remaining on the concave regions; accordingly, the three-dimensional non-woven fabric has the effects of quick permeation and achieves the effects of dryness and comfort. However, improper distribution of the convex regions and the openings is a reason for the convex regions to cause body fluid to leak out of or remain on the non-woven fabric.

### BRIEF SUMMARY OF THE INVENTION

In view of the defects in the prior art products, the present invention aims to provide a soft and dry non-woven fabric having stripe protrusions on a surface thereof, and an absorption product using said non-woven fabric.

To attain the above object, the present invention provides the following technical solutions:
A non-woven fabric, having a surface provided with a plurality of stripe protrusions each extending longitudinally on the non-woven fabric and are arranged in a plurality of columns along a widthwise direction of the non-woven fabric; each of the stripe protrusions has a protruding height of 0.5-3.0 mm; at least one opening region comprising a plurality of openings is at least provided between every two adjacent stripe protrusions; an average aperture size of each of the openings of each opening region is less than 20mm².

The non-woven fabric is a composite non-woven fabric consisting of a surface layer and a bottom layer.

The plurality of stripe protrusions are formed on the surface layer.

The non-woven fabric is one or a combination of hot air through non-woven fabric, spunbond non-woven fabric, spunlace non-woven fabric, or hot rolled non-woven fabric.

Each of the stripe protrusions is a longitudinally continuous stripe protrusion, or each of the stripe projections is constituted by longitudinally discontinuous stripe projections.

Each of the stripe protrusions is solid or hollow.

Each opening region also comprises a plurality of projections.

An absorption product, comprising a fluid-permeable surface layer, an absorbent layer, and a breathable bottom layer; the absorbent layer is arranged between the fluid-permeable surface layer and the breathable bottom layer; the fluid-permeable surface layer of the absorption product is made by the non-woven fabric having the stripe protrusions on the surface thereof.

Beneficial effects of the present invention:
By using the above technical solutions, a surface of the non-woven fabric of the present invention is provided with the stripe protrusions. When the non-woven fabric is used as a surface layer of an absorption product, the stripe protrusions not only reduce the contact area between the surface layer of the absorption product and the human body, but also form an air isolation layer between the human body and the absorption product, thereby effectively prevent skin adhesion to the absorption product and bring softness and comfort to people. The stripe protrusions arranged along a longitudinal direction can guide the body fluid along a longitudinal direction of the absorption product, thereby enlarging the permeation areas of the body fluid, accelerating the infiltration speed of the body fluid, and also effectively preventing side leakage of the body fluid. The opening regions between adjacent stripe protrusions can accelerate the infiltration of the body fluid, and the body fluid can quickly pass through the openings and being absorbed by an absorbent core of the absorption product, thereby improving the dryness of the non-woven fabric.

### BRIEF DESCRIPTION OF THE DRAWINGS

FIG. 1 is a schematic plan view of a non-woven fabric having stripe protrusions on a surface thereof according to Embodiment 1 of the present invention;
FIG. 2 is a schematic cross-sectional view along line O-O' in the non-woven fabric illustrated in FIG. 1;
FIG. 3 is a schematic plan view of a non-woven fabric having stripe protrusions on a surface thereof according to Embodiment 2 of the present invention;
FIG. 4 is a schematic cross-sectional view along line P-P' in the non-woven fabric illustrated in FIG. 3; and
FIG. 5 is a schematic plan view of an absorption product according to Embodiment 3 of the present invention.

Description of the reference numerals used in the figures:

### Embodiment 1:

1- non-woven fabric;
a1- stripe protrusions;
b1-openings.
Embodiment 2:
2- non-woven fabric;
21-surface layer;
22-bottom layer;
a2- stripe protrusions;
b2-openings;
c2-internal space.
Embodiment 3:
3- non-woven fabric;
4-absorption product;
a3- stripe protrusions;
b3- openings.

### DETAILED DESCRIPTION OF THE INVENTION

In order to further explain the technical solutions of the present invention, the present invention will be further described in detail below with reference to some embodiments.

### Embodiment 1

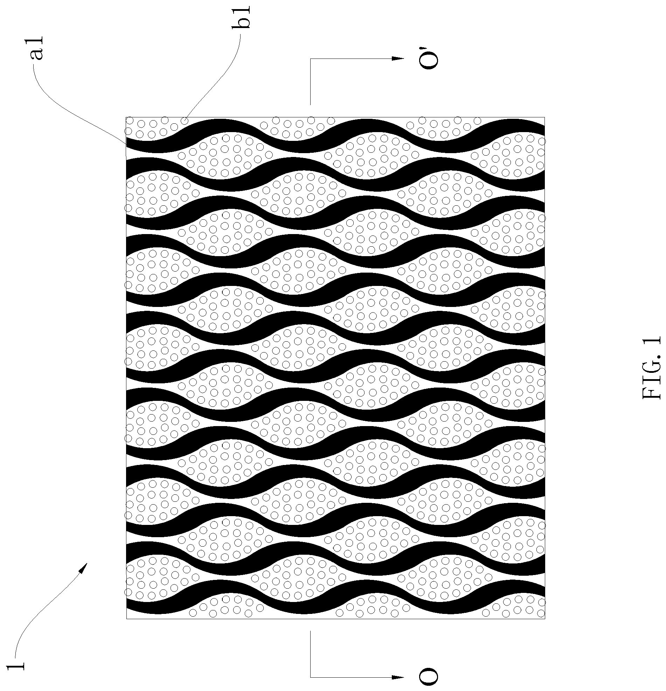

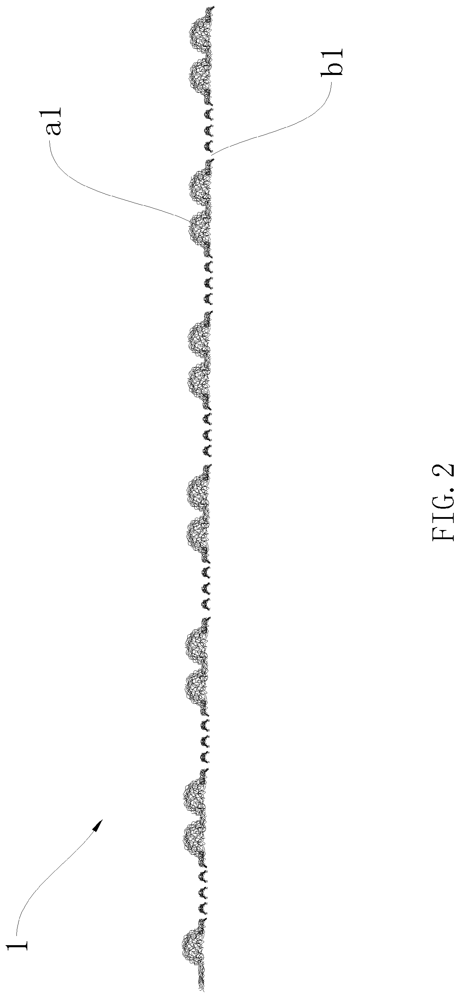

As shown in FIGs. 1 and 2, a non-woven fabric 1 according to this embodiment has a surface provided with a plurality of stripe protrusions a1 each extending longitudinally on the non-woven fabric 1 and are arranged in a plurality of columns along a widthwise direction of the non-woven fabric 1; opening regions each formed by a plurality of openings b1 are at least provided between every two adjacent stripe protrusions a1.

The non-woven fabric 1 in this embodiment is a single-layer hot air through non-woven fabric. Each of the stripe protrusions a1 is a continuous solid stripe protrusion having a protruding height of 0.5-3.0 mm. It should be noted that if the protruding height of the stripe protrusion is too great, user may feel uncomfortable; if the protruding height is too low, the stripe protrusion cannot achieve the function of blocking body fluid. Specifically, each of the stripe protrusions a1 in this embodiment has a protruding height of 0.8 mm; a distance between two adjacent stripe protrusions is 0.5-8 mm; an average aperture size of each opening of each of the opening regions can be less than 20mm². An average aperture size of each opening b1 of each of the opening regions according to this embodiment is 15mm².

By using the above technical solutions, a surface of the non-woven fabric in the present embodiment is provided with the stripe protrusions a1. When the non-woven fabric is used as a surface layer of an absorption product, the stripe protrusions a1 are continuous solid protrusions which are not easy to collapse, and the stripe protrusions a1 arranged along a longitudinal direction can guide the body fluid along a longitudinal direction of the absorption product, thereby enlarging the permeation areas of the body fluid, accelerating the infiltration speed of the body fluid, and also effectively preventing side leakage of the body fluid. The stripe protrusions a1 may be evenly spaced apart from one another by a same distance, or may be unevenly spaced apart from one another by different distances, depending on a width of the absorption product. The opening regions between adjacent stripe protrusions a1 can accelerate the infiltration of the body fluid, and the body fluid can quickly pass through the openings b1 and being absorbed by an absorbent core of the absorption product, thereby improving the dryness of the non-woven fabric.

### Embodiment 2

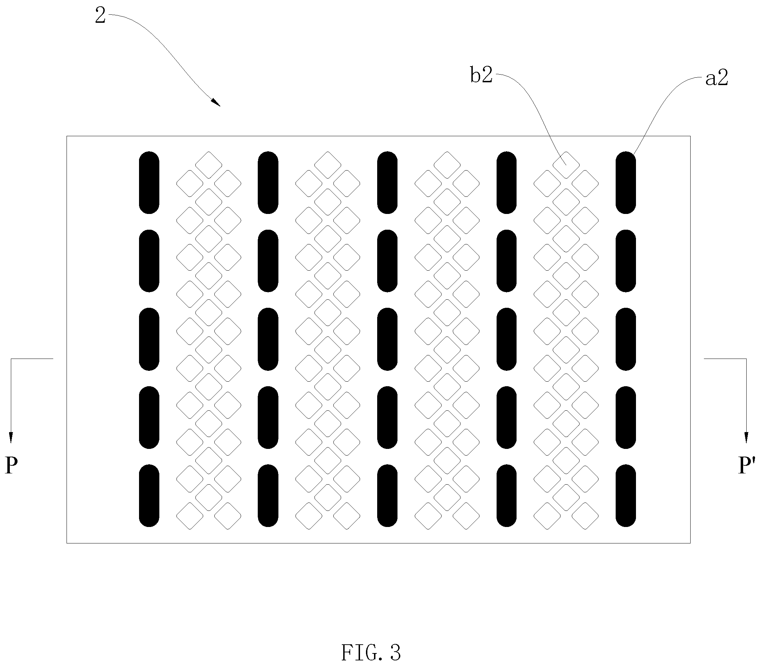

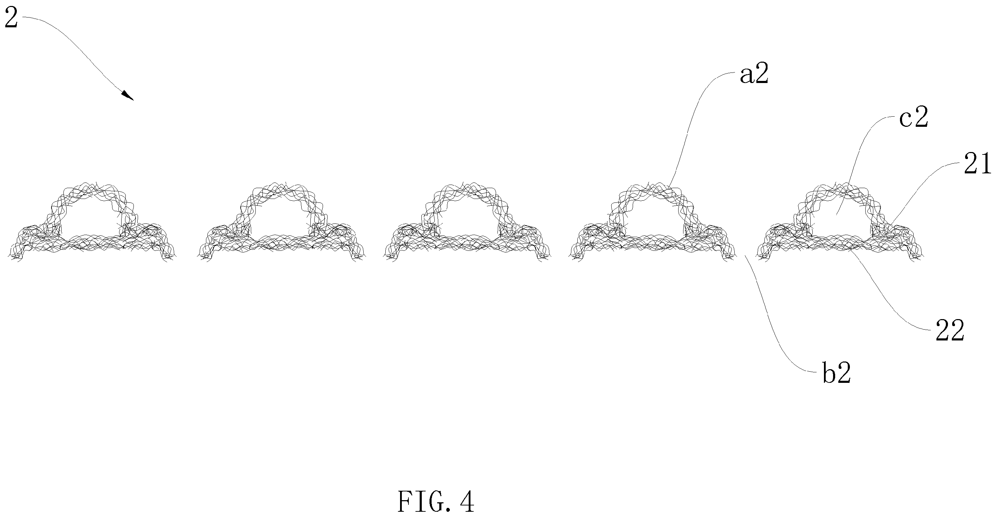

As shown in FIGs. 3 and 4, a non-woven fabric 2 according to this embodiment has a surface provided with a plurality of stripe protrusions a2 each extending longitudinally on the non-woven fabric 2 and are arranged in a plurality of columns along a widthwise direction of the non-woven fabric 2, and an opening region formed by a plurality of openings b2 is provided between every two adjacent stripe protrusions a2.

The non-woven fabric 2 having the stripe protrusions a2 on its surface according to the present embodiment is a composite non-woven fabric consisting of a surface layer 21 and a bottom layer 22; the surface layer 21 is a spunlace non-woven fabric, the bottom layer 22 is a hot air through non-woven fabric; each of the stripe projections a2 is constituted by discontinuous hollow stripe projections each having a protruding height of 0.5-3.0 mm. Each of the discontinuous hollow stripe projections of each of the stripe projections a2 of the present embodiment has a protruding height of 2.5 mm; an average aperture size of each of the openings b2 of each opening region can be less than 20mm², and an average aperture size of each of the openings b2 of each opening region of the present embodiment is 10mm².

By using the above technical solutions, the stripe protrusions a2 in the present embodiment are constituted by discontinuous hollow stripe projections. When the non-woven fabric having such stripe protrusions a2 is used in an absorption product, hollowness of each of the discontinuous hollow stripe projections is formed by an enclosed internal space between the surface layer 21 and the bottom layer 22, so that a breathable channel can be formed to promote internal and external circulation of airflow to reduce the feeling of wetness and stuffiness. The skin-friendly property of the spunlace non-woven fabric of the surface layer 21 can increase the overall softness and comfort of the non-woven fabric, and the bottom layer 22 provides a supporting effect to prevent compressing and collapsing the stripe protrusions a2 and thus prevent the breathable channels from being blocked. Also, the stripe protrusions a2 can also guide the body fluid along a longitudinal direction of the absorption product, enlarge the permeation area of the body fluid, accelerate the infiltration speed of the body fluid, and effectively prevent side leakage of the body fluid.

### Embodiment 3

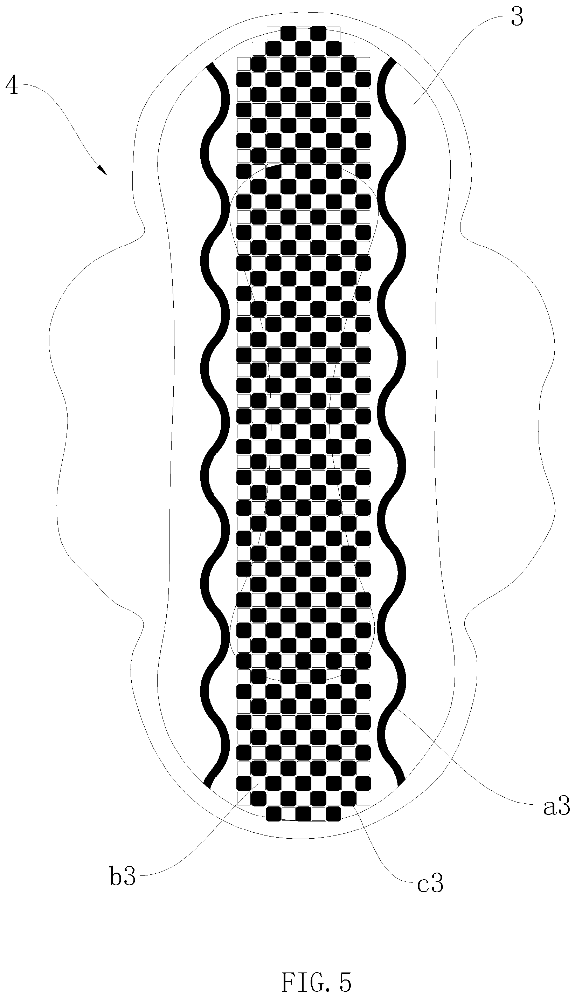

As shown in FIG. 5, an absorption product 4 made at least by a non-woven fabric 3 having stripe protrusions a3 on a surface of the non-woven fabric according to the present embodiment comprises a fluid-permeable surface layer, an absorbent layer, and a breathable bottom layer; the absorbent layer is arranged between the fluid-permeable surface layer and the breathable bottom layer; the fluid-permeable surface layer of the absorption product is made by the non-woven fabric 3 having the stripe protrusions on the surface thereof; a middle region as opposed to two side regions of the absorption product is a permeable region which is mainly used to be in contact with a human body to absorb body fluid, and the two side regions are wing regions of the absorption product which are primarily used to secure the a position of the absorption product. Two sides of the permeation region of the absorption product 4 are arranged with the stripe protrusions a3 of the non-woven fabric 3 respectively to form retaining walls to effectively prevent side leakage of the body fluid. A middle part of the permeation region of the absorption product can also be provided with a plurality of said stripe protrusions a3, so that side leakage prevention can be reinforced, and the body fluid can be guided longitudinally to enlarge an area which the body fluid can permeate along a longitudinal direction of the permeation region, thereby increasing the effective utilization area of the absorption product. The middle part of the penetration region of the absorption product 4 is an opening region which is formed by a plurality of openings b3 and a plurality of projections c3; the opening region is positioned between two adjacent stripe protrusions 3. The openings b3 can accelerate the permeation of the body fluid into the absorbent layer to increase the infiltration speed, and the projections c3 can reduce the contact area between the absorption product and the human body to prevent adhesion to the skin and further improve the dryness.

The above description is only a preferred embodiment of the present invention, and therefore should not be taken as limiting the scope of the present invention, and all variations and modifications achieving equivalent technical effects made within the scope of the present invention and the context of the description should fall within the scope of the present invention.

## Claims

1. A non-woven fabric, having a surface provided with a plurality of stripe protrusions each extending longitudinally on the non-woven fabric and are arranged in a plurality of columns along a widthwise direction of the non-woven fabric; each of the stripe protrusions has a protruding height of 0.5-3.0 mm; at least one opening region comprising a plurality of openings is at least provided between every two adjacent stripe protrusions; an average aperture size of each of the openings of each opening region is less than 20mm².

2. The non-woven fabric of claim 1, wherein the non-woven fabric is a composite non-woven fabric consisting of a surface layer and a bottom layer.

3. The non-woven fabric of claim 2, wherein the plurality of stripe protrusions are formed on the surface layer.

4. The non-woven fabric of claim 1, wherein each opening region also comprises a plurality of projections.

5. The non-woven fabric of claim 1, wherein the non-woven fabric is one or a combination of hot air through non-woven fabric, spunbond non-woven fabric, spunlace non-woven fabric, or hot rolled non-woven fabric.

6. The non-woven fabric of claim 1, wherein each of the stripe protrusions is a longitudinally continuous stripe protrusion, or each of the stripe projections is constituted by longitudinally discontinuous stripe projections.

7. The non-woven fabric of claim 1, wherein each of the stripe protrusions is solid or hollow.

8. An absorption product, comprising a fluid-permeable surface layer, an absorbent layer, and a breathable bottom layer; the absorbent layer is arranged between the fluid-permeable surface layer and the breathable bottom layer; wherein the fluid-permeable surface layer of the absorption product is made by the non-woven fabric having the stripe protrusions on the surface thereof as claimed in any one of claims 1 to 4.
